# EUROPEAN PATENT SPECIFICATION

(11) **EP 3 102 281 B1**
(45) Date of publication and mention of the grant of the patent: **22.01.2020**
(21) Application number: 14808634.1
(22) Date of filing: 08.12.2014
(51) Int. Cl.: A61N 1/32, A61N 1/30, A61N 1/02, A61N 1/04

(54) **IONTOPHORETIC DEVICE WITH INDEPENDENT CURRENT MANAGEMENT**
IONTOPHORETISCHE VORRICHTUNG MIT UNABHÄNGIGER STROMVERWALTUNG
DISPOSITIF IONTOPHORÉTIQUE À GESTION DE COURANT INDÉPENDANTE

(30) Priority: 20.12.2013 FR 1363282
(43) Date of publication of application: 14.12.2016
(73) Proprietor: L'OREAL, 75008 Paris (FR)
(72) Inventor: PLANARD-LUONG, Thi Hong Lien, 91440 Bures sur Yvette (FR)
(74) Representative: L'Oreal
(86) International application number: PCT/EP2014/076824
(87) International publication number: WO 2015/091042

(56) References cited:
- EP-A1- 1 944 057
- EP-A2- 1 046 407
- WO-A1-2013/187951
- US-A1- 2013 253 412

## Description

The present invention relates to devices for carrying out a cosmetic treatment of keratin materials, in particular of the skin, the scalp or the hair.

The expression "cosmetic product" is understood to mean any composition as defined in Council Directive 93/35/EEC of 14 June 1993.

It is known that the application of an electric current to the skin can promote the penetration of active agents.

It is thus known to treat human keratin materials with the aid of iontophoretic devices (J. Singh, K.S. Bhatia, topical iontophoretic drug delivery: pathways, principles, factors and skin irritation, Med. Res. Rev., vol. 16, No. 3, 285-296, 1996).

Iontophoresis allows the diffusion of active agents through the skin by virtue of electrical stimulation in a non-invasive manner. The current applied may be adjustable in terms of intensity and direction (anodal or cathodal). The transcutaneous diffusion of the molecules via iontophoresis is based on two principles, namely electrorepulsion and electroosmosis.

Electrorepulsion is the migration of an ionized molecule by repulsion of charges of the same sign. Thus, if a substance has a positive charge, it will diffuse through the skin at the anode (+).

Electroosmosis is the migration of a molecule, even a non-ionized molecule, by entrainment associated with the flow of water from the anode to the cathode during iontophoresis. The migration is due in particular to the negative charge of the skin. Under the effect of a current, the water or a solvent entrains dissolved substances as it migrates.

The electric current can be applied to the skin by means of an end piece. For large surfaces of the body or of the cheek, the end piece may be large. In areas that are more difficult to access, the end piece may take the form of a small head that is easier to bring into contact or to move.

However, the smaller the size of the end piece, the longer the treatment time. This has an impact on the effectiveness of the care treatment. In order to remedy this problem, some devices that are available on the market are equipped with different end pieces designed for different specific areas of the body.

Thus, the appliance Nu-Skin Galvanic Spa System II ® sold by Nu Skin is provided with a number of end pieces with different shapes. The kit comprises an end piece for the face, an end piece for the body and another for the scalp. Similarly, the patent JP 2012-254168 describes an iontophoretic appliance having a removable end piece. Depending on the area treated, the user can choose an end piece in which the contact surface with the skin is flat or curved.

On the forehead or in certain situations in which the end piece is not pressed properly against the skin as it is being moved, the current intensity risks not being homogeneous over the area treated. For safety reasons, many appliances have limited the current output in order to preempt this problem. This also limits the effectiveness of the treatment.

There is thus a need for a device for cosmetic treatment with an electric current which can ensure a homogeneous current intensity over an area to be treated and which is compact and inexpensive. There is thus a need for a device for cosmetic treatment with an electric current which can be used in complete safety.

To this end, the invention proposes providing a number of electrodes (or pads) on the same end piece and electrically insulating them from one another. Thus, each electrode is managed independently. The intensity of the current at each electrode is adjusted depending on its degree of contact with the skin.

For large surfaces of the body or the cheek, all of the electrodes may be in contact with the skin. In areas that are more difficult to access, it is possible that only some electrodes will be in contact with the skin. Similarly, if the end piece is not pressed properly against the skin while it is being moved, only some electrodes are in contact with the skin.

The device according to the invention makes it possible to detect the electrodes in contact with the skin and to selectively supply only these electrodes with current.

EP1944057 relates to a technique for transdermally administering various ionic drugs by means of iontophoresis and to an iontophoresis device for administering a sleep-inducing agent and a stimulant to a living body while controlling the administered dose and the timing of dose thereof individually.

More specifically, a subject of the invention is a device for the cosmetic treatment of keratin materials with an electric current, comprising:
- an end piece comprising at least two electrodes connected to the same polarities of at least one current generator,
- at least one counter electrode,
- a power supply system,
the electrodes being managed independently of one another by the power supply system.

According to the invention, an *"electrode"* is understood to be a positively charged electrode (anode) or a negatively charged electrode (cathode). This electrode is generally disposed on the external surface of the end piece so as to come into direct contact with the keratin materials. However, the electrode may also be inserted into the external wall of the end piece. In this case, it does not come into direct contact with the keratin materials. In general, the electrode is in contact with the area to be treated.

Throughout the text, the term *"electrode"* means a single insulated electrode. An electrode may be in the form of a ball or pad, for example. A *"counter electrode"* is understood to be a negatively charged electrode (cathode) or a positively charged electrode (anode). The charge of the counter electrode is opposite to that of the electrode. In general, said counter electrode is disposed on the body of the device or on a handpiece. The counter electrode is intended to come into contact with an area of the body of the person undergoing the care treatment. For example, it may be held between the person's fingers. In one configuration, the counter electrode may be disposed on the end piece. If this is the case, it is separated from the electrodes by an insulating space.

A *"power supply system*" is understood to be an electrical assembly that is able to induce a potential difference between the electrodes and the counter electrode. If the end piece is placed on the face and if the counter electrode is held in a hand, the potential difference makes it possible to establish a current between the face and the hand.

The expression *"the electrodes are managed independently of one another by the power supply system*" means that the electrodes are electrically insulated from one another. Each electrode is managed as an independent component. The operation thereof does not depend on that of the other electrodes.

### ELECTRICAL CIRCUIT

According to a first embodiment, the power supply circuit comprises a plurality of current generators that are each connected to an electrode. In this case, each electrode is managed by its own current generator. This embodiment has a simple design.

According to a second embodiment of the invention, the power supply circuit comprises at least one sensor for measuring the current (iₘ) between each electrode and the keratin materials.

Advantageously, the power supply circuit comprises a microcontroller designed to control the voltage between each electrode and the counter electrode, thereby making it possible to control the current. Said voltage is regulated on the basis of the current (iₘ) measured by the sensor.

In this case, the power supply circuit comprises at least one current generator connected to a plurality of electrodes. In other words, the electrodes are connected in parallel to at least one current generator.

Advantageously, the device according to the invention comprises an electronic unit designed to put the power supply of the electrodes on standby if the current detected between an electrode and the skin is below a predetermined threshold value (iₛ). The potential difference between the electrode and counter electrode is kept at a threshold value (Uₛ) by the power supply circuit, with the power supply on standby.

By contrast, if the current (iₘ) detected between an electrode and the skin is above this threshold value (iₛ), the generator increases the current to a predetermined value (I₁) higher than iₛ. The current flows between the electrode and the counter electrode through the skin.

More advantageously, the electronic unit comprises a plurality of current sensors for detecting the current value between each electrode and the skin.

Preferably, the power supply circuit comprises a microcontroller.

Preferably, the threshold current (iₛ) is between 5 µA and 10 µA.

The power supply at each electrode is regulated on the basis of the current measurement (iₘ) detected between the electrode and the skin. This current is advantageously detected by a sensor.

The current (iₘ) is compared with the threshold current value (iₛ) by a microprocessor.

If (iₘ) > (iₛ), then the generator delivers a power supply current at the electrode that ranges up to a predetermined value (I₁).

If (iₘ) < (iₛ), then the generator does not deliver any additional current at the electrode. A standby situation is then the case.

Preferably, the current (I₁) is identified in order to reach a current intensity per unit area that ranges from 0.01 mA/cm² to 0.5 mA/cm², preferably from 0.1 mA/cm² to 0.3 mA/cm².

It will be understood that the term *"microcontroller"* corresponds to a single electronic device, for example a microprocessor chip, or to a set of programmable electronic elements, for example communication gateways that allow management by a third party item of equipment (such as a PC, PDA, etc.).

More preferably, each electrode is connected to a switch. The latter forms a means for detecting the presence of the current. It acts as a current sensor.

Even more advantageously, each electrode is surface mounted on an electronic board.

### ELECTRICAL PARAMETERS

The electrical power source may comprise any non-rechargeable battery or any storage battery. The voltage between the electrodes is for example between 1.2 V and 24 V, preferably between 1.2 and 3.3 V. If appropriate, the passage of the current can create spot heating.

The electrical power source may comprise, for example, a DC voltage source. In a variant, the electrical power source may comprise an electronic circuit for varying the amplitude of the voltage generated over time. This electronic circuit may be a chopper, for example.

At an equivalent current density, the device can in particular deliver a current density, at the skin, of preferably less than or equal to 0.500 mA/cm², for example between 0.1 mA/cm² and 0.500 mA/cm², for example between 0.1 mA/cm² and 0.30 mA/cm².

### ELECTRODES

The electrodes have a visible free surface allowing them to come into direct contact with the skin.

The device can be used in a dabbing manner, the user holding the device in one hand and bringing the electrodes into contact with the skin.

The user can move the device while keeping the electrodes in contact with the skin or move the electrodes away from the skin while the device is being moved between a first area and a second area.

The electrode may be flat, for example in the form of a flat disc or polygon.

The electrode may be porous. The electrodes may have various shapes and for example a surface intended to come into contact with the skin which is convex towards the outside, concave towards the outside, or flat. Preferably, the electrodes are smooth so as not to hurt the skin.

The electrodes may be formed by two spheres or rollers, being able to rotate or not rotate in respective housings.

The electrode may be hollow, being formed for example by stamping or bending an electrically conductive metal sheet.

### Materials able to be used to produce the electrodes

The material(s) forming the electrodes may be identical or different.

At least one electrode may comprise, for example:
- a metal (chromium, stainless steel), for example
- a noble metal (gold, titanium) which is inert with respect to the formulation,
- a metal plated with a noble metal,
- an alloy,
- a composite material (plastics material loaded with carbon microfibres),
- a conductive woven fabric,
- a conductive nonwoven fabric,
- a polymer material rendered conductive,
- a fibrous material,
- conductive polymeric fibres, for example as described in the publication CN101532190,
- carbon fibres, for example as described in the publication JP2009179915,
- silicones rendered conductive by the addition of conductive fillers such as silver, copper or carbon. Such silicones are supplied, for example, by the companies Saint Gobain, Plastics Performance and Aquitaine Caoutchouc 2000,
- conductive metallic fabrics, supplied for example by the companies Utexbel and Cousin Biotech,
- carbon-loaded vinyl, supplied for example by the companies Copema and Rexam,
- electrosurgical plates, supplied for example by the companies Copema and 3M,
- intrinsically conducting polymers, supplied for example by the company Paniplast.

### END PIECE

The end piece may have any geometric shape. The external surface of the end piece forms the application surface in which the electrodes are embedded.

The material may be predominantly in a non-oxidized form.

The application surface may be completely inert from a chemical point of view with respect to the products and keratin materials.

The application surface may be covered with a varnish.

The application surface may be polished.

The end piece may have a biocidal material, for example silver or copper, on its surface. Such a metal may be deposited in the form of a thin layer.

According to one embodiment of the invention, the application surface is smooth. In this case, the device is particularly suitable for wide areas such as the cheeks or the forehead, if a massaging action is not desired.

According to another embodiment of the invention, the end piece has an external surface provided with at least one relief.

In this case, the device may also serve to massage a chosen area of the body.

Advantageously, the relief has at least one boss on which the electrodes are disposed.

The relief element may in particular be chosen from spikes, balls and rollers.

The relief elements may themselves be smooth or have complementary raised areas.

More advantageously, the boss comprises a rotary head (roll-on).

In particular, the head is a ball or a cylinder.

Alternatively, the relief elements may be removable. It is thus possible to change the relief elements of the device, for example in order to modify their dimensions, their surface properties, or else their roughness.

The relief elements may comprise a thermoplastic material of the acrylic type, cellulose type, polycarbonate type, polyamide type, styrene type, polyolefin type, vinyl type or polyethylene terephthalate type and mixtures of said materials in a variable proportion, which are expanded or not expanded. The relief elements may also comprise one or more thermoplastic resins or one or more metals.

The electrodes may be removable, flexible or mounted on ball joints for example in order to better optimize contact with the reliefs of the skin.

### SUPPLEMENTARY FUNCTIONS

The device may comprise one or more treatment modules which can be activated selectively, for example it is conceivable to subject the end piece to light, to a source of heat, or even to vibrations, as will be explained in detail below.

### i) Source of light

According to the invention, the device advantageously comprises a source of light.

The source of light may be, for example, at least one LED, as described in the documents FR-A-2 917 299, US-A-2010/274329 or WO-A-2008/057640.

### ii) Source of heat

According to the invention, the device advantageously comprises a source of heat.

In this case, it is possible to modify the temperature of the external surface of the end piece and/or of the region treated and/or to transmit energy to the external surface of the end piece and/or to the region treated.

The device may comprise for example a heating resistor or a thermoelectric element or an infrared source which is positioned under the end piece.

Preferably, the source of heat comprises an infrared source or a resistor.

The device may comprise a heating module and be configured to heat the external surface of the end piece to a predefined temperature, for example to a temperature of between 35°C and 45°C. In the case of a device comprising a heating module, the heating surface can reach a temperature of 10°C to 35°C greater than room temperature, preferably of 15°C to 25°C greater in heating mode. The power delivered by the heating module may be between 0.25 and 10 W, preferably between 0.5 and 5 W.

More preferably, the source of heat is housed entirely inside the device.

The resistor may be connected to a board by two insulated connectors, using for example the location of the switches.

The infrared source may be integrated into the body of the device, such as the handle. The external part of the device, for example a shell, can serve to guide the infrared radiation towards the end piece.

The electrical circuit may comprise at least one electronic switch which is connected in series with the heating member and makes it possible for example to supply it with power at the desired ratio.

### COSMETIC COMPOSITIONS

It is possible to use at least one cosmetic composition with the device.

The composition(s) used may be in all forms, for example in the form of an aqueous solution, of an oil, of an emulsion, of a powder or of a gel. The composition(s) used may also be sprayed onto the skin.

When the composition(s) used is/are in the form of a gel, the latter can take on the shape of the electrode to which it is applied, as mentioned above.

The composition may be contained in a reservoir.

The reservoir may or may not be removably mounted on the applicator.

Advantageously, the device according to the invention comprises a reservoir of cosmetic product, in particular a removable reservoir.

The reservoir may be for example a sachet, a blister pack, a box, a bottle or a pack made of thermoplastic material.

The composition(s) may comprise an active principle.

Advantageously, the cosmetic product is chosen from:
- a face care or body care composition, comprising in particular an active agent chosen from humectant or moisturizing active agents, anti-ageing active agents, for example depigmenting active agents, active agents that act on cutaneous microcirculation or seboregulating active agents,
- a composition for making up the face or body,
- a hair composition, in particular a composition for washing the hair, for hair care or conditioning, for temporary form retention or shaping of the hair, for the temporary, semi-permanent or permanent dyeing of the hair, or for relaxing or permanent-waving, in particular a composition for relaxing, dyeing or bleaching the roots and hair, and
- a composition for the scalp, in particular an antidandruff composition, a composition for preventing hair loss or for promoting regrowth of the hair, an anti-seborrheic composition, an anti-inflammatory composition, an anti-irritation or soothing composition, a mark-preventing composition or a composition for stimulating or protecting the scalp.

The device can be used in various cosmetic treatments, for example for combating wrinkles, herpes, acne or for redensifying the skin or the hair.

### Brief description of the drawings

Further features and advantages of the present invention will become apparent on reading the following detailed description, which is given with reference to nonlimiting embodiments that are illustrated in the appended drawings, in which:
- Figure 1 illustrates the definitions of the invention in order to better understand the operating principle of the device;
- Figure 2 schematically and partially illustrates the operation of a device according to the invention,
- Figure 3 schematically and partially illustrates an appliance according to the first embodiment of the invention,
- Figure 4 schematically and partially illustrates an appliance according to the second embodiment of the invention,
- Figure 5 shows a block diagram of an electronic board used in a device according to the invention,
- Figure 6 shows a flow chart of a program implemented by the microcontroller in the device according to the invention,
- Figure 7 is a schematic and partially perspective view of an end piece of a device according to the invention,
- Figure 8 is a schematic and partial top view of a ball from Figure 7,
- Figures 9 to 12 illustrate the use of an end piece according to the invention.

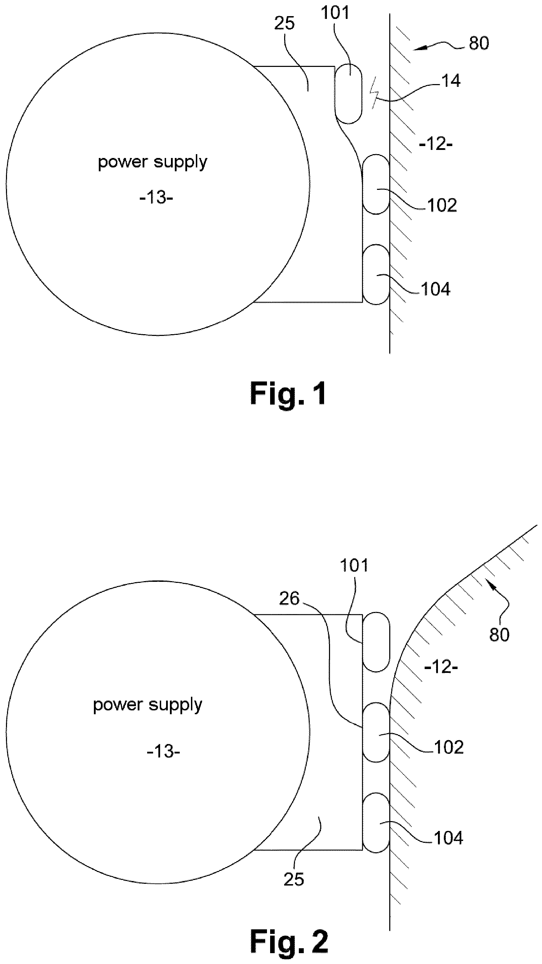

Figure 1 shows a device comprising three electrodes 101, 102 and 104 connected to a power supply 13. Two electrodes 102 and 104 are in contact with the skin 80. The electrode 101 is away from the skin. This electrode 101 is supplied with current in the same way as the electrodes 102 and 104 that are in contact with the skin 80. This power supply of the electrode 101 is symbolized by the flash 14.

In order to use the device, the user can move the end piece over the skin 80 of an area 12 of the body, after optionally having deposited a cosmetic treatment product on the skin or on the electrodes.

Figure 2 shows a device according to the invention, comprising:
- three electrodes 101, 102 and 103 disposed on the external surface of the end piece 25,
- a power supply 13.

This device is used in the same way as the device in Figure 1. However, the operation is different as soon as at least one electrode is no longer in contact with the skin 80.

In the example shown, two electrodes 102 and 104 are fully in contact with the skin 80.

By contrast, for the electrode 101, the surface in contact with the skin is very small. Local heating at the electrode 101 occurs. The current is controlled at this electrode 101 by the power supply system according to the invention.

The electrodes 101, 102 and 103 can be partially inserted into the end piece 25, being for example held inside the latter by various support reliefs which can be produced by moulding thermoplastic material with the casing.

The electrodes have been shown with a contact surface with the skin that is substantially convex, but the invention is not limited to a particular surface shape of an electrode intended to come into contact with the skin.

The electrodes can be balls which are retained by snap-fastening in housings 26 moulded in one piece with the end piece 25.

They may be free to rotate in their housings 26, according to variant embodiments.

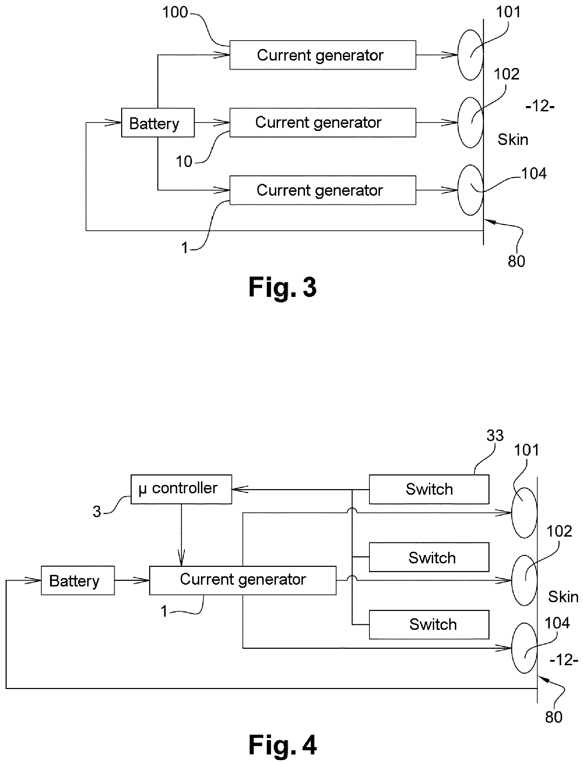

Figure 3 shows a detail of a device according to the first embodiment of the invention. Three generators 1, 10 and 100 output a current with a given power. Three electrodes 101, 102 and 104 are each connected to the current generator 100, 10 and 1, respectively. The current is transmitted to an area of skin 12 to be treated by each electrode 101, 102 and 104. The electrodes 101, 102 and 104 are electrically insulated. They are managed independently of one another so as to adapt the intensity of the current to the number of electrodes which are in contact with the skin. Each electrode 101, 102 and 104 is managed by its associated generator 100, 10 and 1 which is set up to deliver a given current intensity.

The device comprises an electrical circuit which makes it possible to selectively supply the electrodes 101, 102 and 104 with power.

If one electrode is in contact with the skin, the electrical circuit connecting this electrode to the skin is closed. In this case, the power supply at said electrode is provided.

If one electrode is not in contact with the skin, the electrical circuit connecting this electrode to the skin is open. In this case, the power supply at said electrode is not provided.

Figure 4 shows a detail of a device according to the second embodiment of the invention. One generator 1 outputs a current with a given power. The three electrodes 101, 102 and 104 are connected in parallel to the current generator 1. The current is transmitted to an area of skin 80 to be treated by each electrode 101, 102 and 104. The question arises of the effectiveness of the treatment and the homogeneity of the current in the area of skin to be treated, in particular at the periphery of the area. To this end, the device comprises a microcontroller 3 designed to control the voltage between each electrode 101, 102 and 104 and the counter electrode 60 in order to regulate the current generated by the generator. In each loop of the electrode-skin circuit there is placed a switch 33 for detecting the presence of current. This information is communicated to the microcontroller 3.

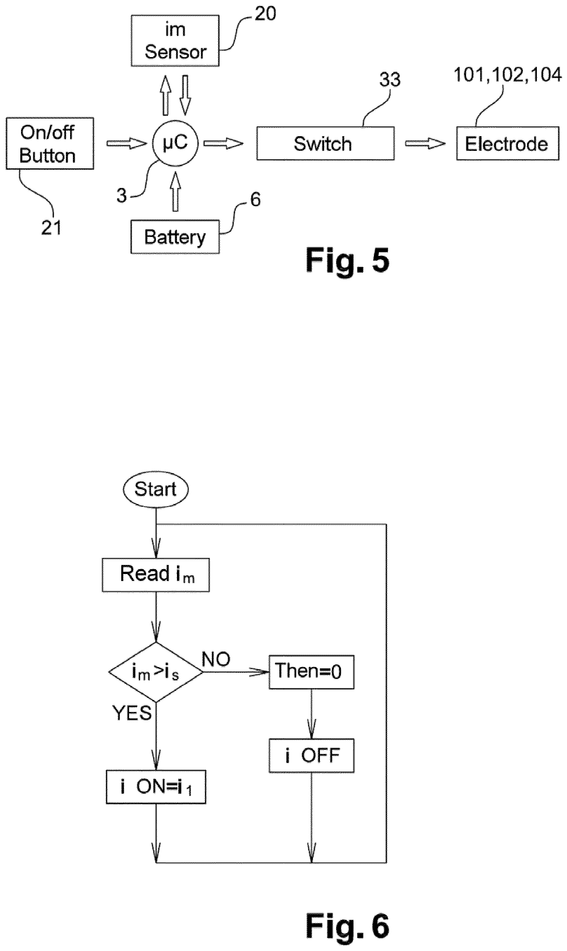

Figure 5 shows a detail of a device according to the second embodiment of the invention. The device comprises the electrodes 101, 102 and 104. A current sensor 20 and a switch are also arranged in the electrical circuit. The microcontroller 3 controls the intensity of the current at the electrodes depending on the measurements provided by the current sensor 20 and on the type of care desired.

The microcontroller 3 is supplied with power by a battery 6 and receives the data measured by the current sensor 20. The microcontroller 3 also receives on/off commands and programming from a button 21 actuated by the user. Such a button 21 may be mechanical or tactile. The microcontroller 3 can also cause the display of information on a screen so that the user can see the operating mode of the device.

Figure 6 shows a flow chart of a program implemented by the microcontroller in the device according to the second embodiment of the invention.

When the device is activated (START) and a program is chosen by the user, the microcontroller collects an item of read data for the current measured (iₘ) from the current sensor.

The current (iₘ) is compared with the threshold current value (iₛ) by a microprocessor.

In the example shown, the current values are fixed as follows:
- iₛ = 10 µA
- the current (I₁) is identified in order to reach a current intensity per unit area that is equal to 0.3 mA/cm².

If (iₘ) > (iₛ), then the generator delivers a power supply current at the electrode that ranges up to a predetermined value (I₁). (i ON = I₁) is then the case at the electrode.

If (iₘ) < (iₛ), then the generator does not deliver any additional current at the electrode. A standby situation is then the case. (i OFF) is then the case at the electrode.

The device may include an indicator light and/or a loudspeaker for informing the user in a sensory manner that the output of current from at least one electrode has been interrupted because the device is being held too far away from the treatment area in a given location.

More specifically, the output power of each electrode may obey a specific regulating law that depends on the location of the electrode in the device (central or peripheral position) and/or that depends on the type of electrode and/or that depends on the current (continuous or pulsed).

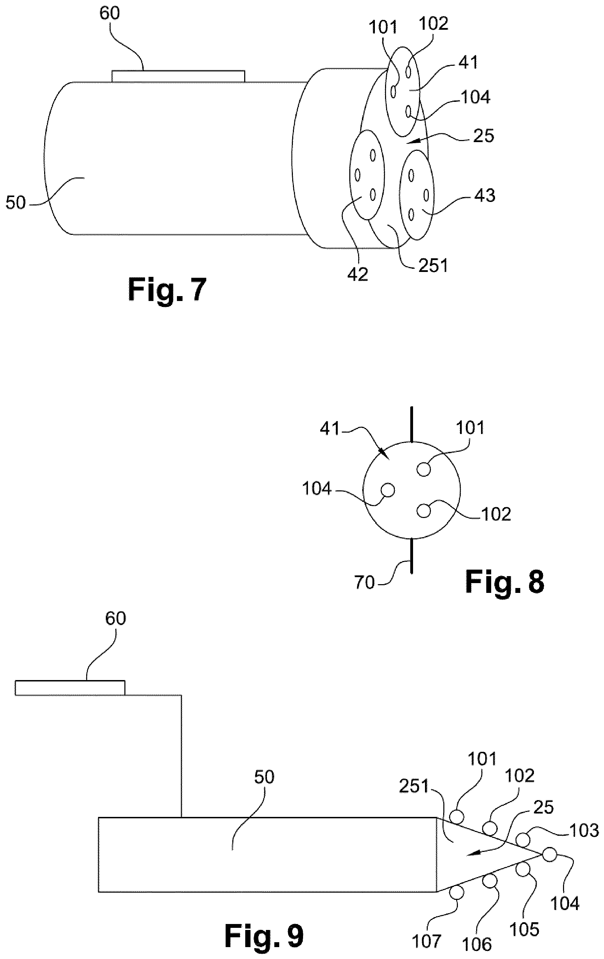

Figure 7 illustrates an end piece 25 integrated into a device according to the invention with a counter electrode 60. The electrodes 101, 102 and 104 may be arranged on mobile balls 41, 42 and 43. For example, the balls may be free to rotate about a core integral with the external surface of the end piece 25. The device may operate according to the first embodiment of the invention (Figure 3) or according to the second embodiment of the invention (Figure 4). The electrodes may constitute components of the "surface mounted components" type. The device has a body 50 made of a shell on which the end piece 25 is mounted. The shell of the body 50 houses other electronic components and a battery 6. The end piece 25 may carry an electronic board on which the current sensor 20 mentioned with reference to Figure 6 and the microcontroller 3 are arranged.

Any type of program can be envisaged in the scope of this invention for controlling pulsed and/or continuous and/or alternate emissions of current. Figure 8 shows a ball 41 of the device from Figure 7 mounted on a core 70 fixed to the end piece 25. In this representation, the core 70 is parallel to the external surface 251 of the end piece 25.

Figures 9 to 12 illustrate the use of a device according to the invention.

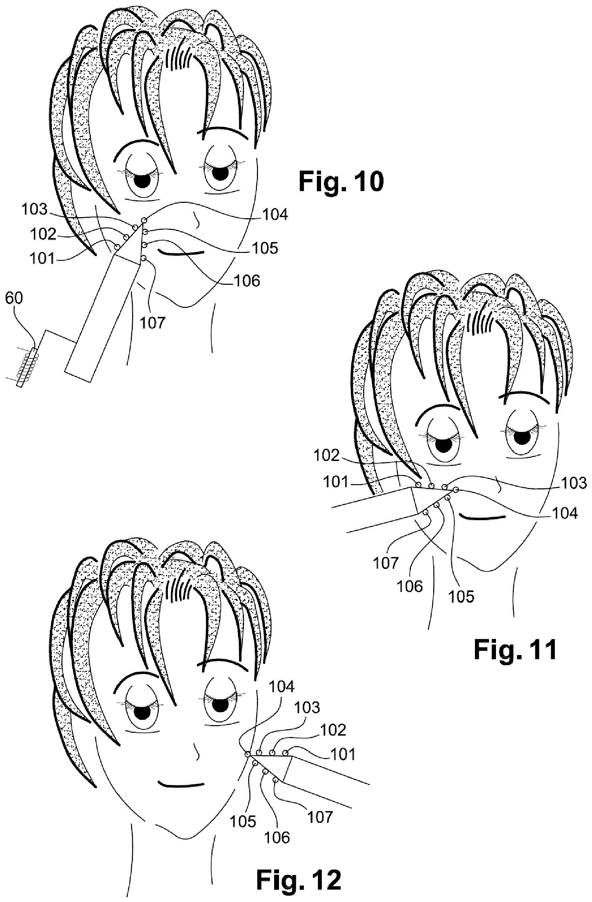

The device used is shown in Figure 9. It comprises an end piece 25 in the form of a nib. Electrodes 101, 102, 103, 104, 105, 106 and 107 are disposed on the external surface 251 of the end piece 25. This device can be used for example at home. The user holds the body 50 with one hand. The counter electrode 60 is held in the other hand. The user applies the end piece 25 to the face (Figure 10). The electrodes which are in contact with the face are represented by empty circles. The electrodes which are not in contact with the face are represented by solid circles.

In Figure 10, all of the electrodes 101, 102, 103, 104, 105, 106 and 107 are in contact with the face. The current passes through at each of these electrodes. The user moves the end piece over her face in the direction of the nose for example (Figure 11). The electrodes 101, 102, 105, 106 and 107 are no longer in contact with the skin. The current is interrupted at these electrodes. It continues to pass through at the electrodes 103 and 104. The user moves the end piece towards the bottom of the cheek (Figure 12). The electrodes 101, 102, 103, 105, 106 and 107 are no longer in contact with the skin. The current is interrupted at these electrodes. It continues to pass through only at the electrode 104.

The present invention has been described with reference to particular embodiments that are illustrated in Figures 1 to 12, and with reference to particular examples, but it will be understood that further variants may be envisaged by a person skilled in the art, in particular the number and types of electrodes may vary and other arrangements than those described may be envisaged in order to form appliances according to the invention. In particular, the shape of the end piece may vary or the position of the electrodes and the counter electrode. The device may for example have an angled shape such that the longitudinal axis X of the body 50 makes an angle with the axis Y of the part that carries the electrodes, the angle between the axes X and Y being 30° for example.

The expression "comprising a" should be understood as being synonymous with "comprising at least one", unless specified to the contrary.

## Claims

1. Device for the cosmetic treatment of keratin materials with an electric current, comprising:
- an end piece (25) comprising at least two electrodes (101, 102) connected to the same polarities of at least one current generator, wherein the end piece has an external surface (251) provided with at least one relief (41,42,43), wherein said relief has at least one boss (41,42,43) on which the electrodes (101,102) are disposed, wherein the boss (41) comprises a rotary head,
- at least one counter electrode (60),
- a power supply system (13),
the electrodes (101, 102) being managed independently of one another by the power supply system (13).

2. Device according to Claim 1, wherein the power supply system (13) comprises a plurality of current generators (10, 100) that are each connected to an electrode (101, 102).

3. Device according to Claim 1, wherein the power supply circuit (13) comprises at least one sensor (20) for measuring the current (iₘ) between each electrode (101, 102) and the keratin materials.

4. Device according to Claim 3, wherein the power supply circuit (13) comprises a microcontroller (3) designed to control the voltage between each electrode (101, 102) and the counter electrode (60), said voltage being regulated on the basis of the current (iₘ) measured by the sensor (20).

5. Device according to either of Claims 3 and 4, wherein each electrode is connected to a switch (33).

6. Device according to any one of the preceding claims, comprising a source of light.

7. Device according to any one of the preceding claims, comprising a source of heat.

8. Device according to Claim 7, wherein the source of heat comprises an infrared source or a resistor.

9. Device according to any one of the preceding claims, comprising a reservoir of cosmetic product, in particular a removable reservoir.

10. Device according to Claim 9, wherein the cosmetic product is chosen from:
- a face care or body care composition, comprising in particular an active agent chosen from humectant or moisturizing active agents, anti-ageing active agents, for example depigmenting active agents, active agents that act on cutaneous microcirculation or seboregulating active agents,
- a composition for making up the face or body,
- a hair composition, in particular a composition for washing the hair, for hair care or conditioning, for temporary form retention or shaping of the hair, for the temporary, semi-permanent or permanent dyeing of the hair, or for relaxing or permanent-waving, in particular a composition for relaxing, dyeing or bleaching the roots and hair, and
- a composition for the scalp, in particular an antidandruff composition, a composition for preventing hair loss or for promoting regrowth of the hair, an anti-seborrheic composition, an anti-inflammatory composition, an anti-irritation or soothing composition, a mark-preventing composition or a composition for stimulating or protecting the scalp.

## Patentansprüche

1. Vorrichtung zur kosmetischen Behandlung von Keratinmaterialien mit einem elektrischen Strom, umfassend:
- ein Endstück (25), das mindestens zwei Elektroden (101, 102) umfasst, die mit den gleichen Polaritäten mindestens eines Stromgenerators verbunden sind, wobei das Endstück eine Außenfläche (251) aufweist, die mit mindestens einem Relief (41, 42, 43) ausgestattet ist, wobei das Relief mindestens einen Ansatz (41, 42, 43) aufweist, auf dem die Elektroden (101, 102) angeordnet sind, wobei der Ansatz (41) einen Drehkopf umfasst,
- mindestens eine Gegenelektrode (60),
- ein Stromversorgungssystem (13),
wobei die Elektroden (101, 102) unabhängig voneinander vom Stromversorgungssystem (13) geregelt werden.

2. Vorrichtung nach Anspruch 1, wobei das Stromversorgungssystem (13) eine Vielzahl von Stromgeneratoren (10, 100) umfasst, die jeweils mit einer Elektrode (101, 102) verbunden sind.

3. Vorrichtung nach Anspruch 1, wobei der Stromversorgungskreis (13) mindestens einen Sensor (20) zur Messung des Stroms (iₘ) zwischen jeder Elektrode (101, 102) und den Keratinmaterialien umfasst.

4. Vorrichtung nach Anspruch 3, wobei der Stromversorgungskreis (13) einen Mikrocontroller (3) umfasst, der zur Steuerung der Spannung zwischen jeder Elektrode (101, 102) und der Gegenelektrode (60) konstruiert ist, wobei die Spannung auf Basis des vom Sensor (20) gemessenen Stroms (iₘ) geregelt wird.

5. Vorrichtung nach einem der Ansprüche 3 und 4, wobei jede Elektrode mit einem Schalter (33) verbunden ist.

6. Vorrichtung nach einem der vorhergehenden Ansprüche, umfassend eine Lichtquelle.

7. Vorrichtung nach einem der vorhergehenden Ansprüche, umfassend eine Wärmequelle.

8. Vorrichtung nach Anspruch 7,
wobei die Wärmequelle eine Infrarotquelle oder einen Widerstand umfasst.

9. Vorrichtung nach einem der vorhergehenden Ansprüche, umfassend ein Reservoir mit einem Kosmetikpräparat, insbesondere ein abnehmbares Reservoir.

10. Vorrichtung nach Anspruch 9, wobei das Kosmetikpräparat ausgewählt ist unter:
- einer Gesichtspflege- oder Körperpflegezusammensetzung, insbesondere umfassend einen Wirkstoff, der ausgewählt ist unter Befeuchtungsmitteln oder feuchtigkeitsspendenden Wirkstoffen, Wirkstoffen gegen Alterung, beispielsweise depigmentierende Wirkstoffe, Wirkstoffe, die auf die Hautmikrozirkulation wirken, oder seboregulierende Wirkstoffe,
- einer Makeup-Zusammensetzung für das Gesicht oder den Körper,
- einer Haarzusammensetzung, insbesondere einer Zusammensetzung zum Waschen der Haare, zur Haarpflege oder Konditionierung, zum temporären Halten oder Formen der Haare, zum temporären, halbpermanenten oder permanenten Färben der Haare, oder zur Entspannung oder für eine Dauerwelle, insbesondere einer Zusammensetzung zum Entspannen, Färben oder Bleichen der Wurzeln und Haare, und
- einer Zusammensetzung für die Kopfhaut, insbesondere einer Zusammensetzung gegen Schuppen, einer Zusammensetzung zur Verhinderung von Haarausfall oder zur Förderung des Nachwachsens der Haare, einer antiseborrhoischen Zusammensetzung, einer entzündungshemmenden Zusammensetzung, einer gegen Reizungen wirkenden oder beruhigenden Zusammensetzung, einer Flecken verhindernden Zusammensetzung oder einer Zusammensetzung zur Stimulierung oder zum Schutz der Kopfhaut.

## Revendications

1. Dispositif pour le traitement cosmétique de matières cornées avec un courant électrique, comprenant :
- une pièce terminale (25) comprenant au moins deux électrodes (101, 102) connectées aux mêmes polarités d'au moins un générateur de courant, la pièce terminale possédant une surface externe (251) dotée d'au moins un relief (41, 42, 43), ledit relief comportant au moins une bosse (41, 42, 43) sur laquelle sont disposées les électrodes (101, 102), la bosse (41) comprenant une tête rotative,
- au moins une contre-électrode (60),
- un système d'alimentation électrique (13),
les électrodes (101, 102) étant gérées indépendamment les unes des autres par le système d'alimentation électrique (13).

2. Dispositif selon la revendication 1, dans lequel le système d'alimentation électrique (13) comprend une pluralité de générateurs de courant (10, 100) qui sont chacun connectés à une électrode (101, 102).

3. Dispositif selon la revendication 1, dans lequel le circuit d'alimentation électrique (13) comprend au moins un capteur (20) pour mesurer le courant (iₘ) entre chaque électrode (101, 102) et les matières cornées.

4. Dispositif selon la revendication 3, dans lequel le circuit d'alimentation électrique (13) comprend un microcontrôleur (3) conçu pour commander la tension entre chaque électrode (101, 102) et la contre-électrode (60), ladite tension étant régulée sur la base du courant (iₘ) mesuré par le capteur (20).

5. Dispositif selon l'une ou l'autre des revendications 3 et 4, dans lequel chaque électrode est connectée à un commutateur (33).

6. Dispositif selon l'une quelconque des revendications précédentes, comprenant une source de lumière.

7. Dispositif selon l'une quelconque des revendications précédentes, comprenant une source de chaleur.

8. Dispositif selon la revendication 7, dans lequel la source de chaleur comprend une source infrarouge ou une résistance.

9. Dispositif selon l'une quelconque des revendications précédentes, comprenant un réservoir de produit cosmétique, en particulier un réservoir amovible.

10. Dispositif selon la revendication 9, dans lequel le produit cosmétique est choisi parmi :
- une composition de soin pour le visage ou de soin pour le corps, comprenant en particulier un agent actif choisi parmi des agents actifs humectants ou hydratants, des agents actifs anti-âge, par exemple des agents actifs dépigmentants, des agents actifs qui agissent sur la microcirculation cutanée ou des agents actifs régulateurs de sébum,
- une composition pour maquiller le visage ou le corps,
- une composition capillaire, en particulier une composition pour le lavage des cheveux, pour le soin ou le conditionnement capillaire, pour la mise en forme ou le coiffage temporaire des cheveux, pour la coloration temporaire, semi-permanente ou permanente des cheveux, ou pour le défrisage ou l'ondulation permanente, en particulier une composition pour le défrisage, la coloration ou la décoloration des racines et des cheveux, et
- une composition pour le cuir chevelu, en particulier une composition antipelliculaire, une composition pour prévenir la chute des cheveux ou pour favoriser la repousse des cheveux, une composition antiséborrhéique, une composition anti-inflammatoire, une composition anti-irritation ou apaisante, une composition évitant les marques ou une composition pour stimuler ou protéger le cuir chevelu.
